# FASCICULE DE BREVET EUROPEEN

(11) **EP 1 092 423 B1**
(45) Date de publication et mention de la délivrance du brevet: **13.05.2009**
(21) Numéro de dépôt: 00118605.5
(22) Date de dépôt: 28.08.2000
(51) Int. Cl.: A61Q 19/02, A61K 8/63

(54) **Utilisation de la DHEA ou de ses précurseurs ou dérivés métaboliques comme dépigmentant**
Verwendung von DHEA oder dessen Vorläufern und Metaboliten als Hautdepigmentierungsmittel
Use of DHEA or its precursors and metabolites as skin depigmentation agents

(30) Priorité: 13.10.1999 FR 9912773
(43) Date de publication de la demande: 18.04.2001
(73) Titulaire: L'ORÉAL, 75008 Paris (FR)
(72) Inventeur: De Lacharriere, Oliver, 75015 Paris (FR); Nouveau, Stéphanie, 92100 Boulogne (FR)
(74) Mandataire: Leszczynski, André

(56) Documents cités:
- EP-A- 0 661 038
- EP-A- 0 723 775
- WO-A-97/10255
- WO-A1-97/03676
- FR-A- 2 760 362
- FR-A- 2 777 181
- US-A- 4 542 129
- US-A- 5 824 671
- PATENT ABSTRACTS OF JAPAN vol. 1995, no. 11, 26 décembre 1995 (1995-12-26) & JP 07 196467 A (KANEBO LTD), 1 août 1995 (1995-08-01)
- PATENT ABSTRACTS OF JAPAN vol. 009, no. 331 (C-321), 25 décembre 1985 (1985-12-25) & JP 60 161912 A (KANEBO KK), 23 août 1985 (1985-08-23)
- CHEMICAL ABSTRACTS, vol. 123, no. 7, 14 août 1995 (1995-08-14) Columbus, Ohio, US; abstract no. 75142q, SHINICHI KAWAI ET AL: "Dehydroepiandrosterone inhibits B16 mouse melanoma cell growth by induction or differentiation" XP002141517 & ANTICANCER RESEACH, vol. 15, no. 2, 1995, pages 427-431,
- E.-E. BAULIEU ET AL: "Dehydroepiandrosterone (DHEA), DHEA sulfate and aging: Contribution of the DHEAge study to a sociobiomedical issue" PROCEEDINGS OF THE NATIONAL ACADEMY OF SCIENCES OF USA., vol. 97, no. 8, 11 avril 2000 (2000-04-11), pages 4279-4284, XP002141516 NATIONAL ACADEMY OF SCIENCE. WASHINGTON., US ISSN: 0027-8424
- PATENT ABSTRACTS OF JAPAN & JP 83 37528 A (ADVANCED SUKIN RES KENKYUSHO), 24 décembre 1996 (1996-12-24)
- DATABASE WPI Week 199638, Derwent Publications Ltd., London, GB; Class D21, AN 1996-379248 & JP 08 183726 A (KOSE) 16 Juillet 1996

## Description

La présente invention concerne l'utilisation de la DHEA ou du sulfate de DHEA dans ou pour la fabrication d'une composition pour application topique sur la peau, comme régulateur de la pigmentation de la peau ou de ses phanères, notamment agent dépigmentant et/ou blanchissant de la peau, en particulier dans le traitement des taches pigmentaires.

La couleur de la peau humaine est fonction de différents facteurs et notamment des saisons de l'année, de l'origine ethnique et du sexe, et elle est principalement déterminée par la nature et la concentration de mélanine produite par les mélanocytes. Les mélanocytes sont les cellules spécialisées qui synthétisent la mélanine organisée sous forme d'organelles particuliers, les mélanosomes. En outre, à différentes périodes de leur vie, certaines personnes voient apparaître sur la peau et plus spécialement sur les mains et le décolleté, des taches plus foncées et/ou plus colorées, conférant à la peau une hétérogénéité. Ces taches sont dues aussi à une concentration importante de mélanine dans les kératinocytes situés à la surface de la peau.

Le mécanisme de formation de la pigmentation de la peau, c'est-à-dire de la formation de la mélanine est particulièrement complexe et fait intervenir schématiquement les principales étapes suivantes: Tyrosine ---> Dopa ---> Dopaquinone ---> Dopachrome ---> Mélanine

La tyrosinase (monophénol dihydroxyl phénylalanine : oxygen oxydo-reductase EC 1.14.18.1) est l'enzyme essentielle intervenant dans cette suite de réactions. Elle catalyse notamment la réaction de transformation de la tyrosine en Dopa (dihydroxyphénylalanine) grâce à son activité hydroxylase et la réaction de transformation de la Dopa en dopaquinone grâce à son activité oxydase. Cette tyrosinase n'agit que lorsqu'elle est à l'état de maturation sous l'action de certains facteurs biologiques.

Une substance est reconnue comme dépigmentante si elle agit directement sur la vitalité des mélanocytes épidermiques où se déroule la mélanogénèse et/ou si elle interfère avec une des étapes de la biosynthèse de la mélanine soit en inhibant une des enzymes impliquées dans la mélanogénèse, soit en s'intercalant comme analogue structural d'un des composés chimiques de la chaîne de synthèse de la mélanine, chaîne qui peut alors être bloquée et ainsi assurer la dépigmentation.

Du document JP 8337528 est connu un actif blanchissant de formule générale (1), ladite formule générale comprenant plus particulièrement la prégnénolone.
Du document CHEMICAL ABSTRACTS, vol. 123, no.7, 14 août 1995 (1995-08-14), Colombus, Ohio, US ; abstract no. : 75142q, Shinichi Kawai *et al. :* « Dehydroepiandrosterone inhibits B16 mouse melanoma cell growth by induction or differentiation », on connaît certains effets de la DHEA entre autres sur la production de mélanine lors d'un test effectué sur culture de cellules cancéreuses de souris (*B16 mélanoma Cell line).*

Par ailleurs, le document WO 97/10255 décrit des compositions cosmétiques ou pharmaceutiques comprenant des dérivés d'esters stéroidiens et notamment le salicylate de DHEA, dans un but de régulation de l'atrophie tissulaire.

Enfin, le document WO 97/03676 décrit des compositions pharmaceutiques comprenant de la DHEA, destinées à une application topique percutanée.

Aussi, il subsiste le besoin d'un nouvel agent blanchissant de la peau humaine ou de ses phanères, à action aussi efficace que ceux connus, mais n'ayant pas leurs inconvénients, c'est-à-dire qui soit non irritant, non toxique et/ou non allergisant pour la peau et/ou ses phanères.

Inversement, on sait que, dans la plupart des populations, le maintien d'une coloration constante de la chevelure est une aspiration importante qui peut soit résulter de considérations esthétiques, soit se justifier par le besoin de remédier à une anomalie de pigmentation d'origine pathologique. Il en est ainsi de la canitie, ou blanchiment des cheveux, qui serait liée à une maladie auto-immune, en particulier au vitiligo.

Dans l'état antérieur de la technique, des agents pro-pigmentants par voie topique ont été proposés, soit dans le domaine de la coloration artificielle par apport de colorants exogènes, tels que la DHA, censés donner à la peau ou aux phanères une coloration la plus proche possible de ce qu'elle est naturellement, soit dans le domaine de la coloration naturelle, par stimulation de la mélanogénèse avec ou sans action des UV.

Or, la demanderesse a trouvé de manière inattendue que la DHEA ou de sulfate de DHEA présentent une activité de régulation de la pigmentation de la peau et de ses phanères. En particulier, il a été démontré que la DHEA présente une activité dépigmentante, même à faibles concentrations, sans faire preuve de cytotoxicité. Elle est en outre utilisable, notamment, dans le traitement de la canitie.

La DHEA, ou déhydroépiandrostérone, est un stéroïde naturel produit essentiellement par les glandes corticosurrénales. Elle est connue pour ses propriétés anti-âge, liées à sa capacité à promouvoir la kératinisation de l'épiderme (JP-07 196 467) et à lutter contre l'ostéoporose (US-5,824,671), ou encore dans le traitement des peaux sèches, en raison de son aptitude à augmenter la production endogène et la sécrétion de sébum et à renforcer l'effet barrière de la peau (US-4,496,556). La DHEA est également décrite dans le traitement de l'obésité et du diabète (WO 97/13500). Il a en outre été proposé d'utiliser le sulfate de DHEA contre l'alopécie (JP-60 142 908) et pour traiter différents signes du vieillissement tels que les rides, la perte d'éclat de la peau et le relâchement cutané (EP-0 723 775). Toutefois, il n'a encore jamais été suggéré que la DHEA ou l'un au moins de ses précurseurs ou dérivés pouvaient avoir une activité de régulation de la pigmentation en agissant directement sur la mélanogénèse.

La présente invention a donc pour objet l'utilisation cosmétique de la DHEA ou du sulfate de DHEA dans une composition pour application topique sur la peau ou ses phanères, pour réguler la pigmentation de la peau ou des phanères.

En particulier, lorsque cette composition est appliquée sur la peau, la DHEA ou du sulfate de DHEA peuvent être utilisés comme agents dépigmentants et/ou blanchissants de la peau et/ou pour améliorer l'homogénéité de la couleur de la peau. En variante, lorsque la composition est appliquée sur les phanères, ils peuvent être utiles comme agent pro-pigmentant des phanères, en particulier des cheveux.

L'invention a également pour objet l'utilisation de la DHEA ou du sulfate de DHEA pour la fabrication d'une composition pour application topique sur la peau ou ses phanères, destinée à réguler la pigmentation de la peau ou des phanères.

En particulier, lorsque cette composition est appliquée sur la peau, elle peut être destinée à la dépigmentation de la peau, notamment au traitement des taches pigmentaires, en particulier des lentigo actiniques. En variante, lorsqu'elle est appliquée sur les phanères, elle peut être destinée au traitement de la canitie.

La DHEA utilisable selon l'invention est par exemple disponible auprès de la société SIGMA ou d'AKZO NOBEL.

La composition contenant la DHEA ou du sulfate de DHEA est appropriée à une utilisation topique et elle contient donc un milieu physiologiquement acceptable, c'est-à-dire compatible avec la peau et ses phanères tels que les poils ou les cheveux.

La composition contenant la DHEA ou du sulfate de DHEA est appropriée à une utilisation topique et elle contient donc un milieu physiologiquement acceptable, c'est-à-dire compatible avec la peau et ses phanères tels que les poils ou les cheveux.

Cette composition renferme une quantité de DHEA ou du sulfate de DHEA suffisante pour obtenir l'effet recherché. Elle peut ainsi renfermer de 10⁻⁶ à 10% en poids, avantageusement de 0,1 à 5% en poids, et mieux, environ 1% en poids de DHEA ou du sulfate de DHEA par rapport au poids total de la composition.

Elle peut se présenter sous toutes les formes galéniques normalement utilisées pour une application topique, notamment sous forme d'une solution aqueuse, hydroalcoolique, d'une émulsion huile-dans-eau ou eau-dans-huile ou multiple, d'un gel aqueux, ou d'un produit anhydre liquide, pâteux ou solide.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage. En variante, elle peut être utilisée comme shampooing ou après-shampooing.

De façon connue, la composition selon l'invention peut contenir également les adjuvants habituels dans les domaines cosmétique et dermatologique, tels que des gélifiants hydrophiles ou lipophiles, des actifs hydrophiles ou lipophiles, des conservateurs, des antioxydants, des solvants, des parfums, des charges, des filtres, des pigments, des absorbeurs d'odeur et des matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans des vésicules lipidiques et/ou dans des nanoparticules.

Bien entendu, l'homme du métier veillera à choisir ces éventuels composés complémentaires, actifs ou non actifs, et/ou leur quantité, de telle manière que les propriétés avantageuses de la DHEA ou du sulfate de DHEA ne soient pas, ou sensiblement pas, altérées par l'adjonction envisagée.

Lorsque la composition selon l'invention est une émulsion, la proportion de la phase grasse peut aller de 0,5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-20, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, on peut utiliser notamment les polyols, les vitamines, les agents kératolytiques et/ou desquamants, les agents anti-inflammatoires, les agents apaisants et leurs mélanges. En outre, bien que la DHEA ou de sulfate de DHEA aient à eux seuls une activité dépigmentante justifiant leur utilisation comme seul actif dépigmentant d'une composition blanchissante, on peut également ajouter à la composition selon l'invention d'autres agents dépigmentants, ce qui permet d'utiliser ces derniers à des doses moins élevées. En cas d'incompatibilité, certains au moins des actifs peuvent être incorporés dans des sphérules, notamment des vésicules ioniques ou non-ioniques et/ou des nanoparticules (nanocapsules et/ou nanosphères), de manière à ce que les actifs incompatibles entre eux soient isolés les uns des autres dans la composition.

Selon une forme de réalisation préférée de l'invention, les compositions contenant la DHEA ou du sulfate de DHEA renferment en outre au moins un filtre UV et/ou un autre agent dépigmentant et/ou un agent kératolytique.

Comme filtres UV, on peut citer:
(1) les dérivés de dibenzoylméthane et en particulier le 4-(ter.-butyl) 4'-méthoxy dibenzoylméthane, notamment celui proposé à la vente sous la dénomination commerciale de "PARSOL 1789" par la Société GIVAUDAN, et le 4-isopropyl-dibenzoylméthane, filtre vendu sous la dénomination de "EUSOLEX 8020" par la Société MERCK;
(2) les filtres actifs dans l'UVA dérivés du benzylidène camphre, dont un exemple particulièrement préféré est l'acide benzène 1,4-[di(3-méthylidènecampho 10-sulfonique)] tel que le produit vendu sous le nom MEXORYL SX par le société CHIMEX, décrit notamment dans les demandes de brevets FR-A- 2 528 420 et FR-A- 2 639 347 ;
(3) les filtres actifs dans l'UVB dérivés du benzylidène camphre, et en particulier le 4-méthyl benzylidène camphre, disponible auprès de la société MERCK sous la dénomination commerciale EUSOLEX 6300 ;
(4) les filtres actifs dans l'UV-A du type benzimidazole ou benzoxazole, tels que l'acide 2-phényl benzimidazole 5-sulfonique, disponible auprès de la société MERCK sous la dénomination commerciale EUSOLEX 232 ;
(5) les dérivés de benzophénone qui peuvent être avantageusement choisis dans le groupe constitué par : la 2-hydroxy-4-méthoxy-benzophénone, encore appelé oxybenzone (benzophénone-3) comme le produit vendu sous le nom UVINUL M40 par BASF ; et l'acide 2-hydroxy-4-méthoxy-benzophénone-5-sulfonique, encore appelé sulisobenzone (benzophénone-4) comme le produit vendu sous le nom UVINUL MS 40 par BASF, ainsi que sa forme sulfonate de sodium (benzophénone-5) ;
(6) les dérivés silanes ou les polyorganosiloxanes à groupement benzophénone tels que ceux décrits dans les documents EP-A-0389 377, FR-A-2 657 351 et EP-A-0 655 453 ;
(7) les benzotriazoles et silicones benzotriazoles, de préférence ceux décrits dans les brevets US-4316033, US-4328346, EP-B-0354145, EP-B-0392883 et EP-B-0660701 ;
(8) les dérivés de triazines décrits notamment dans le brevet US4617390 et les demandes de brevet EP-A-087098, EP-A-0517104, EP-A-0570838 et EP-A-0796851, en particulier la 2,4,6-tris[p-(2'-éthylhexyl-1'-oxy-carbonyl)anilino]-1 3,5-triazine vendue notamment sous la dénomination commerciale de UVINUL T 150 par la Société BASF ;
(9) les dérivés de l'acide cinnamique tels que le para-méthoxy cinnamate de 2-éthylhexyle, vendu notamment sous le nom commercial PARSOL MCX par la Société GIVAUDAN ;
(10) les 2-cyano-33-diphénylacrylate d'alkyle et, de manière préférentielle, l'octocrylène vendu sous le nom Uvinul N 539 par la Société BASF ;
(11) le composé de formule (I) suivante, ou 2-(2H-benzotriazole-2-yl)-4-méthyl-6-[2-méthyl-3-[1,3,3,3-tétraméthyl-1-[(triméthylsilyl)oxy] disiloxanyl]propynyl]phénol, décrit dans la demande de brevet EP-A-0 392 883 : 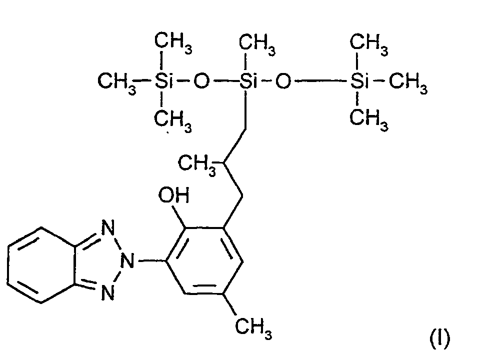
(12) et leurs mélanges.

L'invention a donc également pour objet une composition comprenant dans un milieu physiologiquement acceptable, la DHEA ou du sulfate de DHEA et au moins un agent dépigmentant choisi parmi : l'acide kojique ; l'acide ellagique ; l'arbutine et ses dérivés ; l'hydroquinone ; les dérivés d'aminophénol tels que le N-cholestéryloxycarbonyl-para-aminophénol et le N-éthyloxycarbonyl-para-aminophénol ; les dérivés d'iminophénol ; l'acide L-2-oxothiazolidine-4-carboxylique ou procystéine, ainsi que ses sels et esters ; et les extraits de réglisse, de mûrier et de scutellaire.

Les agents kératolytiques susceptibles d'être utilisés dans les compositions selon invention comprennent notamment les α-hydroxyacides tels que les acides citrique, lactique, glycolique, mandélique, malique et tartrique ; les β-hydroxyacides et notamment l'acide salicylique et ses dérivés décrits dans les demandes FR-A-2 581 542, EP-875 495, WO 98/35973 et EP-756 866; les α-cétoacides et les β-cétoacides ; les rétinoïdes et en particulier le rétinol et les esters de rétinyle ; les inhibiteurs d'HMG-CoA réductase, comme décrit dans la demande EP-738 510 ; et les dérivés de sucre tels que ceux décrits dans la demande EP-853 472, et en particulier le O-octanoyl-6'-β-D-maltose.

Ces compositions constituent notamment des crèmes de protection, de traitement ou de soin pour le visage, pour les mains ou pour le corps, des laits corporels de protection ou de soin, des lotions, gels ou mousses pour le soin ou le traitement de la peau, des lotions de nettoyage ou de désinfection, des compositions pour le bain, des fonds de teint et des crèmes teintées. Dans ces derniers cas, la composition contient des pigments.

L'invention va maintenant être illustrée à l'aide des exemples non limitatifs suivants.

### Exemple 1: Composition à base de DHEA

On a préparé la composition suivante, dans laquelle les proportions des différents constituants sont indiquées en pourcentage pondéral :

| | |
|---|---|
| DHEA | 2 % |
| Isostéarate de propylène glycol | 13 % |
| Polyéthylène glycol (8 OE) | 5 % |
| Propylène glycol | 3 % |
| Pentylène glycol | 3 % |
| Stéarate de glycéryle et stéarate de polyéthylène glycol (100 OE) | 5 % |
| Mono-stéarate de sorbitane oxyéthyléné (20 OE) | 0,5 % |
| Alcool cétylique oxyéthyléné (20 OE) oxypropyléné (5 OP) | 1 % |
| Gélifiants | 0,5 % |
| Benzoates d'alkyle en C₁₂₋₁₅ | 4 % |
| Ethanol | 3 % |
| Hydroxyde de sodium | 0,12% |
| Conservateurs | 0,7 % |
| Eau | qsp 100 % |

### Exemple 2 : Mise en évidence de l'activité dépigmentante de la DHEA

### a- Protocole

On a testé une émulsion huile-dans-eau gélifiée, renfermant 1% en poids de DHEA comme seul actif, sur un groupe de vingt sujets de sexe féminin, âgés de 55 à 70 ans. L'émulsion a été appliquée matin et soir pendant quatre mois sur le dos d'une main.

Les taches présentes sur la peau ont été évaluées avant et après traitement par observation visuelle réalisée par un expert, en attribuant aux taches un score global de 0 à 7 en fonction de leur nombre, de leur taille et de leur intensité (évaluation clinique).

Elles ont également été évaluées par colorimétrie, à l'aide d'un chromamètre Minolta CR200 (évaluation biophysique). Cet appareil décrit les couleurs dans un espace à trois dimensions, en utilisant le système de coordonnées L, a* et b* recommandé par la CIE, où L* exprime la luminance ou clarté de la couleur (0 correspondant au blanc et 100 au noir), a* est la composante de séparation entre le vert (négatif) et le rouge (positif) et est corrélée à l'érythème cutané, et b* varie du bleu (négatif) au jaune (positif) et exprime la pigmentation cutanée. Ce paramètre b* a été utilisé pour évaluer l'évolution des taches au cours du traitement. Il est la résultante de la moyenne de six mesures individuelles effectuées par zone et par temps de mesure.

### b- Résultats

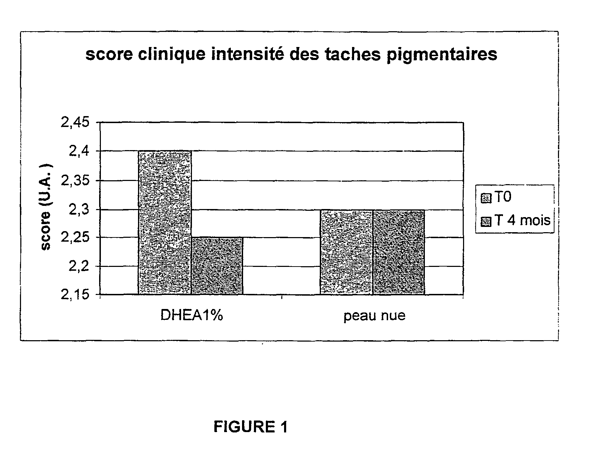

Evaluation clinique : le graphe illustré à la Figure 1 montre une amélioration du score clinique global des taches pigmentaires sur la main traitée avec la DHEA alors que ce score est constant sur la main non traitée.

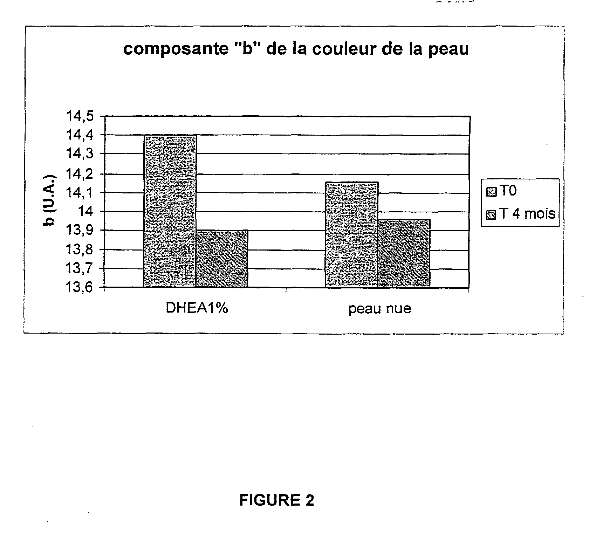

Evaluation biophysique : le graphe illustré à la Figure 2 montre une diminution de la composante b de la couleur de la peau, donc une réduction de la pigmentation de la peau, plus importante sur la main traitée à la DHEA que sur la main non traitée.

## Revendications

1. Utilisation cosmétique de la DHEA ou du sulfate de DHEA dans une composition pour application topique sur la peau ou ses phanères, pour réguler la pigmentation de la peau ou des phanères.

2. Utilisation cosmétique de la DHEA ou du sulfate de DHEA dans une composition pour application topique sur la peau, comme agent dépigmentant et/ou blanchissant de la peau et/ou pour améliorer l'homogénéité de la couleur de la peau.

3. Utilisation cosmétique de la DHEA ou du sulfate de DHEA dans une composition pour application topique sur les phanères, comme agent pro-pigmentant des phanères, en particulier des cheveux.

4. Utilisation de la DHEA ou du sulfate de DHEA pour la fabrication d'une composition pour application topique sur la peau ou ses phanères, destinée à réguler la pigmentation de la peau ou des phanères.

5. Utilisation de la DHEA ou du sulfate de DHEA pour la fabrication d'une composition pour application topique sur la peau destinée à la dépigmentation de la peau.

6. Utilisation de la DHEA ou de sulfate de DHEA pour la fabrication d'une composition pour application topique sur la peau, destinée au traitement des taches pigmentaires, en particulier des lentigo actiniques.

7. Utilisation de la DHEA ou du sulfate de DHEA pour la fabrication d'une composition pour application topique sur les phanères, destinée au traitement de la canitie.

8. Utilisation selon l'une quelconque des revendications précédentes, **caractérisée en ce que** la DHEA ou le sulfate de DHEA est présent en une quantité allant de 10⁻⁶ à 10% en poids, par rapport au poids total de la composition.

9. Utilisation selon la revendication 8, **caractérisée en ce que** la DHEA ou le sulfate de DHEA est présent en une quantité allant de 0,1 à 5% en poids, par rapport au poids total de la composition.

10. Utilisation selon la revendication 9, **caractérisée en ce que** la DHEA ou le sulfate de DHEA est présent en une quantité d'environ 1% en poids, par rapport au poids total de la composition.

11. Utilisation selon l'une quelconque des revendications précédentes, **caractérisée en ce que** ladite composition renferme en outre au moins un filtre UV et/ou un autre agent dépigmentant et/ou un agent kératolytique.

12. Utilisation selon la revendication 11, **caractérisée en ce que** ledit filtre UV est choisi parmi : les dérivés de dibenzoyiméthane et en particulier le 4-(ter.-butyl) 4'-méthoxy dibenzoylméthane et le 4-isopropyl-dibenzoylméthane ; les filtres actifs dans l'UVA dérivés du benzylidène camphre, tels que l'acide benzène 1,4-[di(3-méthylidènecampho 10-sulfonique)] ; les filtres actifs dans l'UVB dérivés du benzylidène camphre, et en particulier le 4-méthyl benzylidène camphre ; les filtres actifs dans l'UV-A du type benzimidazole ou benzoxazole, tels que l'acide 2-phényl benzimidazole 5-suifonique ; les dérivés de benzophénone notamment choisis dans le groupe constitué par : la 2-hydroxy-4-méthoxy-benzophénone, l'acide 2-hydroxy-4-méthoxy-benzophénone-5-sulfonique, et sa forme sulfonate de sodium ; les dérivés silanes ou les polyorganosiloxanes à groupement benzophénone ; les benzotriazoles et silicones benzotriazoles ; les dérivés de triazines, en particulier la 2,4,6-tris[p-(2'-éthylhexyl-1'-oxy-carbonyl)anilino]-1,3,5-triazine ; les dérivés de l'acide cinnamique tels que le para-méthoxy cinnamate de 2-éthylhexyle ; les 2-cyano-33-diphénylacrylate d'alkyle et, de manière préférentielle, l'octocrylène ; le composé de formule (I) suivante, ou 2-(2H-benzotriazole-2-yl)-4-méthyl-6-[2-méthyl-3-[1,3,3,3-tétraméthyl-1-[(triméthylsilyl)oxy]disiloxanyl]propynyl]phénol : 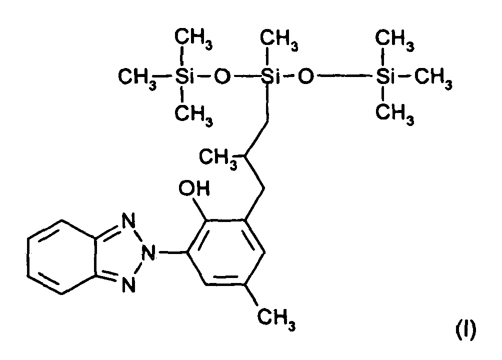 et leurs mélanges.

13. Utilisation selon la revendication 11, **caractérisée en ce que** ledit autre agent dépigmentant est choisi parmi : l'acide kojique ; l'acide ellagique ; l'arbutine et ses dérivés ; l'hydroquinone ; les dérivés d'aminophénol tels que le N-cholestéryloxycarbonyl-para-aminophénol et le N-éthyloxycarbonyl-para-aminophénol ; les dérivés d'iminophénol ; l'acide L-2-oxothiazolidine-4-carboxylique ou procystéine, ainsi que ses sels et esters ; l'acide ascorbique et ses dérivés, notamment le glucoside d'ascorbyle ; et les extraits de plantes, en particulier de réglisse, de mûrier et de scutellaire.

14. Utilisation selon la revendication 11, **caractérisée en ce que** ledit agent kératolytique est choisi parmi : les α-hydroxyacides tels que les acides citrique, lactique, glycolique, mandélique, malique et tartrique ; les β-hydroxyacides et notamment l'acide salicylique et ses dérivés ; les α-cétoacides et les β-cétoacides ; les rétinoïdes et en particulier le rétinol et les esters de rétinyle ; les inhibiteurs d'HMG-CoA réductase ; et les dérivés de sucre tels que le O-octanoyl-6'-β-D-maltose.

15. Composition comprenant, dans un milieu physiologiquement acceptable, la DHEA ou le sulfate de DHEA et au moins un agent dépigmentant choisi parmi : l'acide kojique ; l'acide ellagique ; l'arbutine et ses dérivés ; l'hydroquinone ; les dérivés d'aminophénol tels que le N-cholestéryloxycarbonyl-para-aminophénol et le N-éthyloxycarbonyl-para-aminophénol ; les dérivés d'iminophénol; l'acide L-2-oxothiazolidine-4-carboxylique ou procystéine, ainsi que ses sels et esters ; et les extraits de réglisse, de mûrier et de scutellaire.

## Claims

1. Cosmetic use of DHEA or DHEA sulfate in a composition for topical application to the skin or its integuments, to regulate the pigmentation of the skin or the integuments.

2. Cosmetic use of DHEA or DHEA sulfate in a composition for topical application to the skin, as a depigmenting and/or bleaching agent for the skin and/or to improve the homogeneity of the colour of the skin.

3. Cosmetic use of DHEA or DHEA sulfate in a composition for topical application to the integuments, as a pro-pigmenting agent for the integuments, in particular the hair.

4. Use of DHEA or DHEA sulfate for the manufacture of a composition for topical application to the skin or its integuments, which is intended to regulate the pigmentation of the skin or the integuments.

5. Use of DHEA or DHEA sulfate for the manufacture of a composition for topical application to the skin which is intended for depigmenting the skin.

6. Use of DHEA or DHEA sulfate for the manufacture of a composition for topical application to the skin, which is intended for treating pigmentation marks, in particular actinic lentigo.

7. Use of DHEA or DHEA sulfate for the manufacture of a composition for topical application to the integuments, which is intended for the treatment of canities.

8. Use according to any one of the preceding claims, **characterized in that** the DHEA or DHEA sulfate is present in an amount ranging from 10⁻⁶ % to 10% by weight, relative to the total weight of the composition.

9. Use according to Claim 8, **characterized in that** the DHEA or DHEA sulfate is present in an amount ranging from 0.1% to 5% by weight, relative to the total weight of the composition.

10. Use according to Claim 9, **characterized in that** the DHEA or DHEA sulfate is present in an amount of about 1% by weight, relative to the total weight of the composition.

11. Use according to any one of the preceding claims, **characterized in that** the said composition also contains at least one UV screening agent and/or one other depigmenting agent and/or one keratolytic agent.

12. Use according to Claim 11, **characterized in that** the said UV screening agent is chosen from: dibenzoylmethane derivatives and in particular 4-(tert-butyl)-4'-methoxydibenzoylmethane and 4-isopropyldibenzoylmethane; benzylidenecamphor-based UVA-active screening agents such as benzene-1,9-bis(3-methylidenecamphor-10-sulfonic acid); benzylidenecamphor-based UVB-active screening agents, and in particular 4-methyl-benzylidenecamphor; benzimidazole-type or benzoxazole-type UVA-active screening agents, such as 2-phenylbenzimidazole-5-sulfonic acid; benzophenone derivatives chosen in particular from the group consisting of: 2-hydroxy-4-methoxybenzophenone, 2-hydroxy-4-methoxybenzophenone-5-sulfonic acid, and its sodium sulfonate form; silane derivatives or polyorganosiloxanes containing a benzophenone group; benzotriazoles and benzotriazole silicones; triazine derivatives, in particular 2,4,6-tris[p-(2'-ethylhexyl-1'-oxycarbonyl)anilino]-1,3,5-triazine; cinnamic acid derivatives such as 2-ethylhexyl para-methoxycinnamate; alkyl 2-cyano-3,3-diphenylacrylate, and preferably octocrylene; the compound of formula (I) below, or 2-(2H-benzotriazol-2-yl)-4-methyl-6-[2-methyl-3-[1,3,3,3-tetramethyl-1-[(trimethylsilyl)-oxy]disiloxanyl]propynyl]phenol: 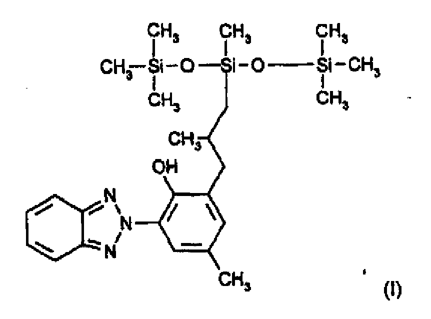 and mixtures thereof.

13. Use according to Claim 11, **characterized in that** the said other depigmenting agent is chosen from: kojic acid; ellagic acid; arbutin and its derivatives; hydroquinone; aminophenol derivatives such as N-cholesteryloxycarbonyl-para-aminophenol and N-ethyloxycarbonyl-para-aminophenol; iminophenol derivatives; L-2-oxothiazolidone-4-carboxylic acid or procysteine, as well as its salts and esters; ascorbic acid and its derivatives, in particular ascorbyl glucoside; and plant extracts, in particular extract of liquorice, of mulberry and of skullcap.

14. Use according to Claim 11, **characterized in that** the said keratolytic agent is chosen from: α-hydroxy acids such as citric acid, lactic acid, glycolic acid, mandelic acid, malic acid and tartaric acid; β-hydroxy acids and in particular salicylic acid and its derivatives; α-keto acids and β-keto acids; retinoids and in particular retinol and retinyl esters; HMG-CoA reductase inhibitors; and sugar derivatives such as O-octanoyl-6'-β-D-maltose.

15. Composition comprising, in a physiologically acceptable medium, DHEA or DHEA sulfate and at least one depigmenting agent chosen from: kojic acid; ellagic acid; arbutin and its derivatives; hydroquinone; aminophenol derivatives such as N-cholesteryloxycarbonyl-para-aminophenol and N-ethyloxycarbonyl-para-aminophenol; iminophenol derivatives; L-2-oxothiazolidone-4-carboxylic acid or procysteine, as well as its salts and esters; and extracts of liquorice, of mulberry and of skullcap.

## Patentansprüche

1. Kosmetische Verwendung des DHEA oder des Sulfats von DHEA in einer Zusammensetzung zur topischen Anwendung auf der Haut oder ihren Phaneren zur Regulierung der Pigmentierung der Haut oder der Phaneren.

2. Kosmetische Verwendung des DHEA oder des Sulfats von DHEA in einer Zusammensetzung zur topischen Anwendung auf der Haut, als Depigmentierungsmittel und/oder Bleichmittel der Haut und/oder zur Verbesserung der Gleichmäßigkeit der Farbe der Haut.

3. Kosmetische Verwendung des DHEA oder des Sulfats von DHEA in einer Zusammensetzung zur topischen Anwendung auf den Phaneren, als Pro-Pigmentierungsmittel der Phaneren, insbesondere der Haare.

4. Kosmetische Verwendung des DHEA oder des Sulfats von DHEA zur Herstellung einer Zusammensetzung zur topischen Anwendung auf der Haut oder ihren Phaneren, die zur Regulierung der Pigmentierung der Haut oder der Phaneren bestimmt ist.

5. Kosmetische Verwendung des DHEA oder des Sulfats von DHEA zur Herstellung einer Zusammensetzung zur topischen Anwendung auf der Haut, die zur Depigmentierung der Haut bestimmt ist.

6. Kosmetische Verwendung des DHEA oder des Sulfats von DHEA zur Herstellung einer Zusammensetzung zur topischen Anwendung auf der Haut, die zur Behandlung der Pigmentflecken, insbesondere der aktinischen Lentigines, bestimmt ist.

7. Verwendung des DHEA oder des Sulfats von DHEA zur Herstellung einer Zusammensetzung zur topischen Anwendung auf den Phaneren, die zur Behandlung der Canities bestimmt ist.

8. Verwendung nach einem der vorhergehenden Ansprüche, **dadurch gekennzeichnet, dass** das DHEA oder das Sulfat von DHEA in einer Menge vorhanden ist, die von 10⁻⁶ bis 10 Gew.-% reicht, bezogen auf das Gesamtgewicht der Zusammensetzung.

9. Verwendung nach Anspruch 8, **dadurch gekennzeichnet, dass** das DHEA oder das Sulfat von DHEA in einer Menge vorhanden ist, die von 0,1 bis 5 Gew.-% reicht, bezogen auf das Gesamtgewicht der Zusammensetzung.

10. Verwendung nach Anspruch 9, **dadurch gekennzeichnet, dass** das DHEA oder das Sulfat von DHEA in einer Menge von etwa 1 Gew.-% vorhanden ist, bezogen auf das Gesamtgewicht der Zusammensetzung.

11. Verwendung nach einem der vorhergehenden Ansprüche, **dadurch gekennzeichnet, dass** die Zusammensetzung weiterhin mindestens einen UV-Filter und/oder ein anderes Depigmentierungsmittel und/oder ein keratolytisches Mittel einschließt.

12. Verwendung nach Anspruch 11, **dadurch gekennzeichnet, dass** der UV-Filter ausgewählt ist aus: Dibenzoylmethanderivaten, und insbesondere 4-(tert.-Butyl)-4'-methoxydibenzoylmethan und 4-Isopropyldibenzoylmethan; aktiven Filtern in den UVA-Derivaten von Benzylidencampher, wie 1,4-Di(3-methylidencampher-10)benzolsulfonsäure; aktiven Filtern in den UVB-Derivaten von Benzylidencampher und insbesondere 4-Methylbenzylidencampher; aktiven Filtern in den UV-A-Derivaten von Typ eines Benzimidazols oder Benzoxazols, wie 2-Phenylbenzimidazol-5-sulfonsäure, Benzophenonderivaten, die insbesondere aus der Gruppe ausgewählt sind, bestehend aus: 2-Hydroxy-4-methoxybenzophenon, 2-Hydroxy-4-methoxybenzophenon-5-sulfonsäure und ihrer Natriumsulfonatform; Silanderivaten oder den Polyorganosiloxanen mit Benzophenongruppe; Benzotriazolen und Siliconbezotriazolen; Derivaten von Triazinen, insbesondere 2,4,6-Tris[p-(2'-ethylhexyl-1'-oxycarbonyl)anilino]-1,3,5-triazin; Zimtsäurederivaten, wie Paramethoxy-2-ethylhexylcinnamat; 2-Cyano-33-diphenylalkylacrylat, und bevorzugt Octocrylen; der Verbindung der folgenden Formel (I) oder 2-(2H-Benzotriazol-2-yl)-4-methyl-6-[2-methyl-3-[1,3,3,3-tetramethyl-1-[(trimethylsilyl)oxyldisiloxanyl]propinyl]phenol- 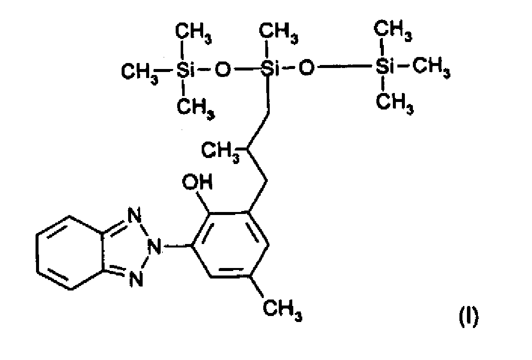 und ihren Gemischen.

13. Verwendung nach Anspruch 11, **dadurch gekennzeichnet, dass** das andere Depigmentierungsmittel ausgewählt ist aus: Koijsäure, Ellaginsäure, Arbutin und seinen Derivaten, Hydrochinon; Aminophenolderivaten, wie N-Cholesteryloxycarbonyl-para-aminophenol und N-Ethyloxycarbonyl-para-aminophenol; Iminophenolderivaten; L-2-Oxothiazolidin-4-carbonsäure oder Procystein sowie ihren Salzen und Estern; Ascorbinsäure und ihren Derivaten, insbesondere Ascorbylglucosid; und Extrakten von Pflanzen, insbesondere von Süßholz, Maulbeerbaum und Helmkraut.

14. Verwendung nach Anspruch 11, **dadurch gekennzeichnet, dass** das keratolytische Mittel ausgewählt ist aus: α-Hydroxysäuren, wie Citronensäure, Milchsäure, Glycolsäure, Mandelsäure, Äpfelsäure, Weinsäure; β-Hydroxysäuren und insbesondere Salicylsäure und ihren Derivaten; α-Ketosäuren und β-Ketosäuren; Retinoiden und insbesondere Retinol und Retinylestem; HMG-CoA-Reduktase-Inhibitoren; und Zucker-Derivaten, wie O-Octanoyl-6'-β-D-maltose.

15. Zusammensetzung, umfassend, in einem physiologisch verträglichen Medium, DHEA oder das Sulfat von DHEA und mindestens ein Depigmentierungsmittel, das ausgewählt ist aus: Koijsäure, Ellaginsäure, Arbutin und seinen Derivaten, Hydrochinon; Aminophenolderivaten, wie N-Cholesteryloxycarbonyl-para-aminophenol und N-Ethyloxycarbonyl-para-aminophenol; Iminophenolderivaten; L-2-Oxothiazolidin-4-carbonsäure oder Procystein sowie ihren Salzen und Estern; und den Extrakten von Süßholz, Maulbeerbaum und Helmkraut.
